# FASCICULE DE BREVET EUROPEEN

(11) **EP 2 712 652 B1**
(45) Date de publication et mention de la délivrance du brevet: **26.05.2021**
(21) Numéro de dépôt: 13186397.9
(22) Date de dépôt: 27.09.2013
(51) Int. Cl.: A61M 39/10

(54) **Système de connexion médicale anti-dévissage**
Medizinisches Verbindungssystem mit Aufschraubblockierung
Anti-unscrewing medical connection system

(30) Priorité: 28.09.2012 FR 1259159
(43) Date de publication de la demande: 02.04.2014
(73) Titulaire: Vygon, 95440 Ecouen (FR)
(72) Inventeur: Carrez, Jean-Luc, 95440 Ecouen (FR); Coussegal, Jean-Louis, 95250 Beauchamp (FR); Barre, Laurent, 95440 Ecouen (FR); Guyomarc'h, Pierrick, 95120 Ermont (FR); Algrain, Isabelle, 95380 Puiseux en France (FR)
(74) Mandataire: Regimbeau

(56) Documents cités:
- WO-A1-00/62844
- US-A- 5 292 308
- US-A1- 2008 172 039
- US-A1- 2012 157 914

## Description

### Domaine de l'invention

L'invention concerne de manière générale un système de connexion médicale, adapté pour connecter un premier dispositif médical et un deuxième dispositif médical.

### Arrière-plan de l'invention

Les cathéters et circuits associés, comprenant des moyens de connexion par vissage, sont susceptibles de se dévisser de manière intempestive, que ce soit lorsqu'ils sont en place, notamment sur un patient, ou au cours de leur transport. Les circuits peuvent être très simples, et correspondre notamment à une seringue connectée sur un cathéter, ou complexes, comme c'est le cas des circuits d'oncologie permettant la perfusion simultanée ou séquencée de plusieurs médicaments au moyen de pompes, de poches ou encore de chambres goutte à goutte et/ou gravité. Or le dévissage accidentel de ces circuits peut avoir de lourdes conséquences, comme créer des entrées d'air jusqu'au patient, arrêter des perfusions, déconnecter des dispositifs médicaux, fausser des thérapies, etc.

Par exemple, le document US 2012/0157914 décrit un connecteur comprenant un raccord distal, un carter, un raccord proximal, et un élément d'étanchéité. Le raccord distal est fixé à l'aide de filetages sur le carter tandis que le raccord proximal est fixé par encliquetage à l'aide de languette. Néanmoins, on note que dans ce document le dévissage accidentel de l'un et ou l'autre des embouts qui sont vissés sur les raccords est possible, que ce soit volontairement ou accidentellement.

Le document WO 00/62844 décrit également un connecteur comprenant un raccord distal, un carter, un raccord proximal, et un élément d'étanchéité. Le raccord distal est fixé au carter par vissage et est maintenu dans ce carter par collage d'un anneau de retenue, tandis que le raccord proximal peut être mobile en rotation dans le carter ou être fixé au carter à l'aide de filetages. Ici encore, la déconnexion d'un embout vissé sur l'un et/ou l'autre des raccords est possible, volontairement ou accidentellement.

Enfin, le document US 2008/172039 décrit un connecteur médical, comprenant un raccord proximal adapté pour être connecté à un embout fileté, un carter adapté pour loger le raccord proximal et comprenant des moyens de blocage en rotation du raccord proximal seulement dans le sens de vissage de l'embout, et un raccord distal, mobile en rotation dans le carter et dans lequel est inséré en force un conduit. Ce connecteur est susceptible d'éviter le dévissage de l'embout fileté. Néanmoins, la rotation dans le sens de vissage de cet embout fileté peut déloger le conduit du carter, et donc permettre son retrait du connecteur.

Par ailleurs, un mauvais montage d'un circuit peut entrainer des problèmes de fonctionnement, comme une impossibilité de purge pour chasser complètement l'air, des problèmes de réductions de débit, de volumes morts trop grands ou mal connus, ou encore des pertes de produits médicamenteux très coûteux, etc.

On a donc proposé d'assembler les circuits directement chez les fabricants d'accessoires de cathétérisme, plutôt qu'au niveau des opérateurs. Mais là encore des risques de dévissage existent, notamment lors des manipulations de conditionnement, de stérilisation, de transports, ou encore de déconditionnement. La responsabilité du système est donc remontée au niveau du fabricant, plutôt que de l'opérateur. Les fabricants ont donc été amenés à réaliser des collages au niveau des connections, afin de renforcer le vissage. Le collage est toutefois coûteux et limite les choix possibles pour les matériaux utilisables. En effet, un matériau susceptible d'être collé doit présenter des propriétés bien précises, telles que la résistance chimique, la transparence, la sensibilité thermique, etc. qui dépendent du type de collage choisi.

### Résumé de l'invention

Un objectif de l'invention est donc de proposer un système de connexion médicale, permettant de connecter un ou plusieurs dispositifs médicaux par vissage, qui soit capable d'empêcher les dévissages accidentels du ou des dispositifs qui y sont connectés, quels que soient les matériaux utilisés pour les dispositifs médicaux, et qui soit en outre de coût modéré et simple à réaliser.

De manière optionnelle, l'invention vise également à empêcher tout dévissage du ou des dispositifs médicaux, et à servir le cas échéant d'adaptateur afin de transformer une connexion par vissage d'un type donné en une connexion d'un type différent, sans risque de démontage ultérieur.

A cet effet, l'invention propose un système de connexion médicale, conforme à la revendication 1.

Certaines caractéristiques optionnelles mais non limitatives du système de connexion sont les suivantes :
- les moyens de blocage en rotation comprennent au moins une encoche réalisée dans une paroi interne du carter, adaptée pour coopérer avec une dent s'étendant depuis le premier raccord,
- la ou chaque dent fait saillie le long de l'axe longitudinal d'une surépaisseur annulaire s'étendant le long d'une périphérie de l'embout fileté, et présente une première arête sensiblement parallèle à l'axe longitudinal et une seconde arête, s'étendant de manière continue entre une première extrémité libre de la première arête et la surépaisseur,
- la ou chaque encoche est définie par un renfoncement annulaire de la paroi interne du carter qui s'étend transversalement par rapport à l'axe longitudinal, et comprend une première arête sensiblement parallèle à l'axe longitudinal et une deuxième arête, s'étendant de manière continue entre une extrémité de la première arête et un bord annulaire dudit renfoncement,
- le carter est dimensionné de sorte que, lorsque le premier raccord et le deuxième raccord sont logés dans le carter, seul l'embout fileté du premier raccord et du deuxième raccord est susceptible de faire saillie en dehors du carter, de sorte que le premier raccord et le deuxième raccord ne présentent pas de surface susceptible d'être saisie par un opérateur,
- le premier embout de connexion est adapté pour être inséré dans le deuxième embout de connexion, et les moyens d'encliquetage comprennent une collerette et un appui en regard s'étendant respectivement du premier embout de connexion et du deuxième embout de connexion, adaptés pour venir en prise lorsque le premier embout de connexion est inséré dans le deuxième embout de connexion,
- le premier raccord comprend un premier embout de connexion adapté pour être inséré dans un deuxième embout de connexion du deuxième raccord, et le système de connexion comprend en outre un organe d'étanchéité disposé entre le premier embout de connexion et le deuxième embout de connexion, et
- le premier raccord et le deuxième raccord comprennent des repères visuels servant d'aide au positionnement relatif du raccord mâle (10) et du raccord femelle par rapport au carter.

### Brève description des dessins

D'autres caractéristiques, buts et avantages de la présente invention apparaîtront mieux à la lecture de la description détaillée qui va suivre, faite en regard des figures annexées données à titre d'exemples non limitatifs et sur lesquelles :

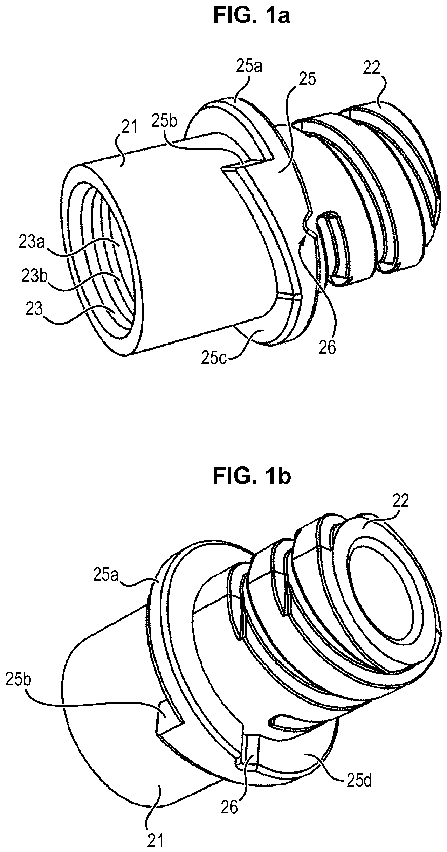

La figure 1a est une vue de face en perspective d'un exemple de réalisation d'un premier raccord d'un système de connexion médicale conforme à l'invention,
La figure 1b est une vue de derrière en perspective du premier raccord de la figure 1a,

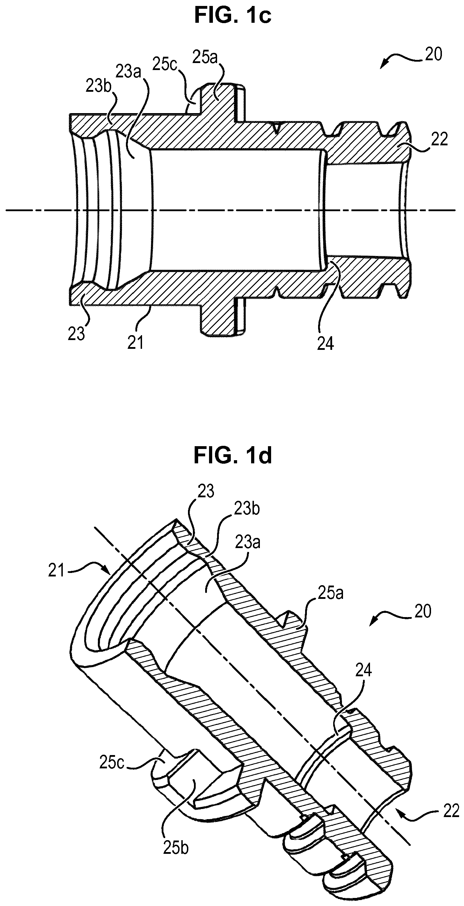

La figure 1c est une vue en coupe transversale du premier raccord de la figure 1a,
La figure 1d est une vue en coupe transversale en perspective du premier raccord de la figure 1a,

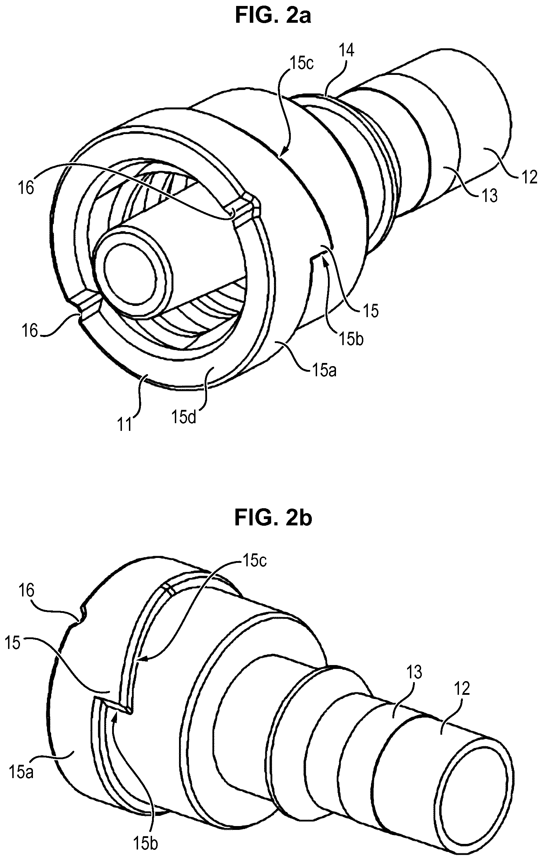

La figure 2a est une vue de face en perspective d'un exemple de réalisation d'un deuxième raccord d'un système de connexion médicale conforme à l'invention,
La figure 2b est une vue de derrière en perspective du deuxième raccord de la figure 1a,

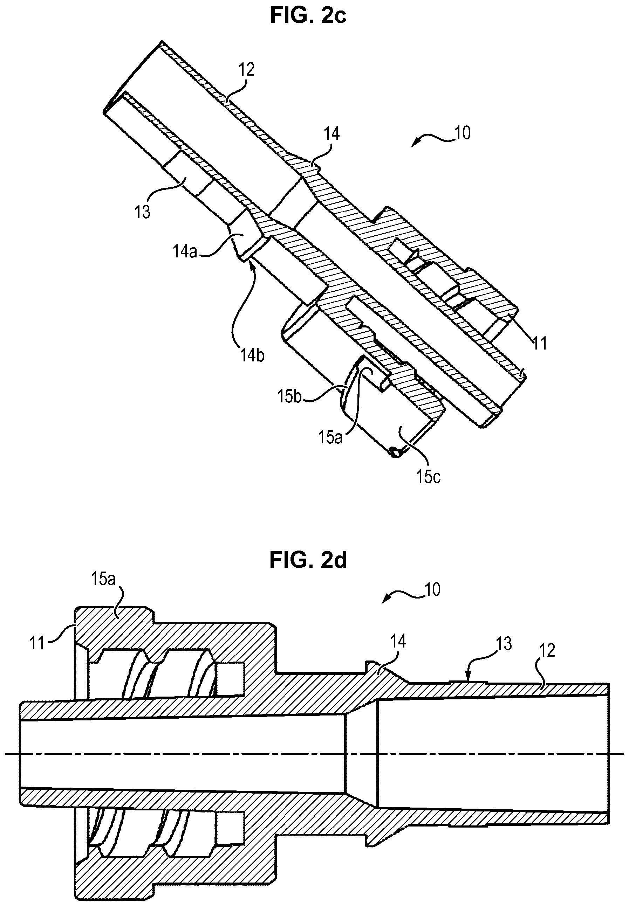

La figure 2c est une vue en coupe transversale en perspective du deuxième raccord de la figure 1a,
La figure 2d est une vue en coupe transversale du deuxième raccord de la figure 1a,

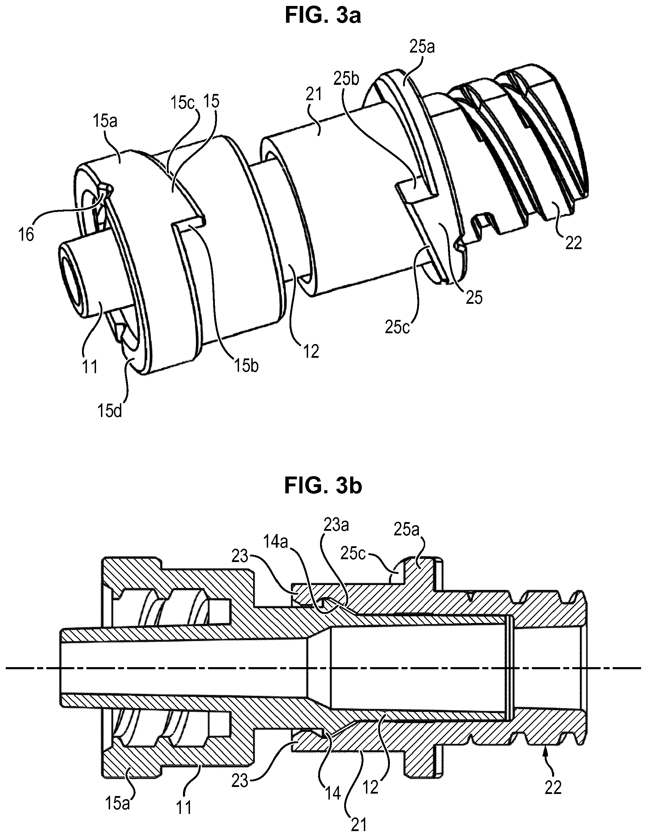

La figure 3a est une vue de côté en perspective du premier raccord des figures 1a-1d et du deuxième raccord des figures 2a-2d assemblés,
La figure 3b est une vue en coupe transversale de la figure 3a,

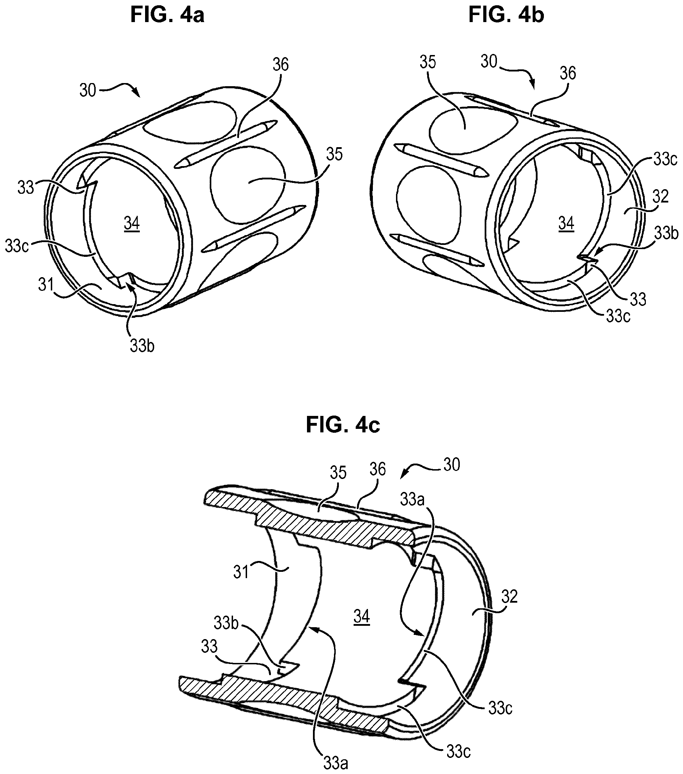

La figure 4a est une première une vue de face en perspective d'un exemple de réalisation d'un carter d'un système de connexion médicale conforme à l'invention,
La figure 4b est une deuxième une vue de face en perspective du carter de la figure 4a,
La figure 4c est une vue en coupe en perspective de la figure 4b,
La figure 5a est une vue de côté en perspective d'un exemple de système de connexion médicale conforme à l'invention,
La figure 5b est une vue de face en perspective du système de connexion médicale de la figure 5a,
La figure 5c est une vue en coupe du système de connexion médicale de la figure 5a, dans une première position de vissage, et
La figure 5d est une vue en coupe du système de connexion médicale de la figure 5a, dans une deuxième position de vissage.

### Description détaillée d'un mode de réalisation

Un exemple de réalisation d'un système de connexion médicale 1 conforme à l'invention va à présent être décrit en relation avec les figures annexées, dans le cadre d'un système de connexion fluidique, c'est-à-dire un système permettant de mettre en communication fluidique avec étanchéité un premier dispositif médical avec un deuxième dispositif médical. Ceci n'est cependant pas limitatif, dans la mesure où le système peut être mis en oeuvre dans le cadre d'une connexion sans étanchéité.

Un tel système de connexion médicale 1 comprend :
- un premier raccord,
- un deuxième raccord, et
- un carter 30.

Dans le cadre d'un système de connexion fluidique, le premier raccord peut par exemple être un raccord mâle 10, tandis que le deuxième raccord peut être un raccord femelle 20.

Le raccord mâle 10 définit un passage coaxial avec un axe longitudinal X, et comprend un embout distal fileté 11 ainsi qu'un embout proximal.

Le raccord femelle 20 quant à lui définit un passage coaxial avec cet axe longitudinal X, et comprend un embout proximal fileté 22, ainsi qu'un embout distal.

Chacun des embouts filetés 11, 22 des raccords mâle 10 et femelle 20 est adapté pour être connecté à un dispositif médical respectif, comprenant des moyens de connexion par vissage complémentaires. Par exemple, il peut s'agir de connexions normalisées de type Luer Lock. Comme nous le verrons plus tard, le sens de vissage de l'embout distal 11 du raccord mâle 10 est opposé au sens de vissage de l'embout proximal 22 du raccord femelle 20.

Par exemple, sur les figures, l'embout distal 11 du raccord mâle 10 est de type Luer Lock mâle, tandis que l'embout proximal 22 du raccord femelle 20 est de type Luer Lock femelle. Ceci n'est cependant pas limitatif, d'autres types de connexion par vissage pouvant être utilisés. Par ailleurs, l'embout distal 11 du raccord mâle 10 peut être un embout femelle, tandis que l'embout proximal 22 du raccord femelle 20 peut être un embout mâle. Les embouts filetés distal 11 et proximal 22 peuvent en outre être tous deux mâles ou femelles.

Nous verrons par la suite que seul le sens de vissage des embouts 11, 22 présente une importance.

Dans ce qui suit, « proximal » désignera une partie (par exemple un embout) qui se situe à proximité d'un opérateur, tandis que « distal » désignera une partie qui est éloignée de l'opérateur. Sur les figures, l'opérateur se situe sur le côté droit.

L'embout proximal 12 du raccord mâle 10 présente une forme globalement tubulaire, et est adapté pour être introduit, de préférence avec étanchéité, dans l'embout distal correspondant du raccord femelle 20. Pour cela, un diamètre externe de l'embout proximal 12 du raccord mâle 10 est sensiblement égal au diamètre interne de l'embout distal 21 du raccord femelle 20.

L'étanchéité de la connexion du raccord mâle 10 et du raccord femelle 20 peut être améliorée en interposant entre l'embout proximal 12 du raccord mâle 10 et l'embout distal 21 du raccord femelle 20 un organe d'étanchéité 13, adapté pour être comprimé localement entre les deux embouts 12, 21. Par exemple, l'embout proximal 12 du raccord mâle 10 peut comprendre une surépaisseur locale d'étanchéisation 13, pouvant par exemple être annulaire. En variante, l'embout proximal 12 du raccord mâle 10 est lisse, la surépaisseur d'étanchéisation étant réalisée sur la face interne de l'embout distal 21 du raccord femelle 20. Dans les deux formes de réalisation, la surépaisseur 13 peut être formée intégralement avec l'embout concerné, ou rapportée.

La forme tubulaire de l'embout proximal 12 du raccord mâle 10 et de l'embout distal 21 du raccord femelle 20 permet en outre de guider le raccord mâle 10 lors de son introduction dans le raccord femelle 20.

Le raccord mâle 10 et le raccord femelle 20 comprennent en outre des moyens de couplage 14, 23, permettant leur connexion mutuelle sans empêcher leur rotation relative. Plus précisément, les moyens de couplage 14, 23 comprennent des moyens d'encliquetage 14, 23 agencés de manière à permettre l'introduction puis le blocage du raccord mâle 10 dans le raccord femelle 20, empêchant ainsi toute séparation ultérieure des raccords 10, 20, et à autoriser la rotation relative du raccord mâle 10 et du raccord femelle 20.

Par exemple, les moyens d'encliquetage 14, 23 peuvent comprendre un appui 23 réalisé dans une surface interne de l'embout distal 21 du raccord femelle 20, adapté pour coopérer avec une collerette 14 faisant saillie d'une surface externe de l'embout proximal 12 du raccord mâle 10.

La collerette 14 peut présenter une section transversale de forme globalement triangulaire, avec une pente inclinée 14a en direction proximale, interrompue de manière à former un bord droit 14b transversal. De la sorte, l'épaisseur de la collerette 14 augmente progressivement en direction distale jusqu'à son bord droit 14b, facilitant ainsi l'introduction de l'appui 23 grâce à la pente 14a formant une surface de guidage, puis sa retenue grâce à son bord droit 14b.

L'appui 23 peut être disposé au niveau de l'extrémité libre de l'embout distal 21 du raccord femelle 20, et former le cas échéant une surépaisseur annulaire.

Lors de la connexion du raccord mâle 10 et du raccord femelle 20, l'embout proximal 12 du raccord mâle 10 est alors introduit dans l'embout distal 21 du raccord femelle 20, et guidé dans le raccord femelle 20 le long de l'axe longitudinal X jusqu'à ce que l'appui 23 du raccord femelle 20 se trouve en butée contre la pente inclinée 14a de la collerette 14. En poussant sur le raccord mâle 10 en direction du raccord femelle 20, l'appui 23 a alors tendance à s'écarter 30 légèrement, ce qui lui permet de glisser le long de la pente inclinée 14a à mesure que le raccord mâle 10 est enfoncé dans le raccord femelle 20, jusqu'à arriver au niveau du bord droit 14b transversal. L'appui 23 se rabat alors en aval de la collerette 14. Il n'est alors plus possible de séparer les deux raccords 10, 20. En effet, en tirant sur le raccord mâle 10 et le raccord femelle 20 dans des directions opposées, l'appui 23 vient en butée contre le bord droit 14b de la collerette 14, et ne peut pas s'écarter 30 de lui-même pour la franchir (puisqu'il n'y a pas, dans ce sens, de pente inclinée 14a).

Afin de renforcer le couplage des raccords 10, 20 et de faciliter le passage de la collerette 14, la paroi de l'embout distal 21 du raccord femelle 20 peut être localement amincie en amont de l'appui 23, dans une zone 23b adjacente de celui-ci, de manière à augmenter l'élasticité de l'extrémité libre de l'embout et faciliter l'écartement de l'appui 23 lors du passage de la collerette 14. Lors du couplage de raccord mâle 10 et du raccord femelle 20, la collerette 14 vient alors se loger contre la partie 23b localement amincie de la paroi de l'embout proximal 21.

Dans le but d'empêcher toute translation du raccord mâle 10 par rapport au raccord femelle 20, l'embout proximal 12 du raccord mâle 10 et l'embout distal 21 du raccord femelle 20 peuvent comprendre des moyens formant butée, adaptés pour coopérer ensemble une fois le raccord mâle 10 couplé au raccord femelle 20 à l'aide des moyens d'encliquetage 14, 23.

Dans la forme de réalisation illustrée sur les figures, ce rôle de butée est joué par les moyens d'encliquetage 14, 23 eux-mêmes. En effet, la paroi de l'embout distal 21 peut présenter, en amont de la partie amincie, une partie inclinée 23a adaptée pour coopérer avec la pente inclinée 14a de la collerette 14. De la sorte, une fois le raccord mâle 10 est introduit et positionné dans le raccord femelle 20, l'appui 23 est positionné en aval de la collerette 14, qui est logée dans la gorge générée par l'amincissement local 23b de la paroi, de sorte que la pente inclinée 14a de la collerette 14 se trouve positionnée en regard de la paroi inclinée 23a. En poussant sur le raccord mâle 10 en direction du raccord femelle 20, la paroi inclinée 23a vient donc en butée contre la pente inclinée 14a de la collerette 14, ce qui limite leur rapprochement le long de l'axe longitudinal X ; à l'inverse, en tirant sur le raccord mâle 10 dans la direction opposée au raccord femelle 20, l'appui 23 vient en butée contre le bord droit 14b de la collerette 14, limitant ainsi l'écartement relatif des deux raccords 10, 20. Tout mouvement de translation est donc limité, voire empêché. Un jeu fonctionnel peut cependant exister permettant une translation relative négligeable des raccords 10, 20, pouvant par exemple dépendre des tolérances dimensionnelles des raccords 10, 20.

De manière alternative ou en plus, ce rôle de butée peut être joué par le contact entre l'extrémité proximale de l'embout proximal 12 du raccord mâle 10 et une surépaisseur 24 de la paroi interne de l'embout distal 21 du raccord femelle 20.

Le carter 30 comprend une paroi globalement tubulaire, adaptée pour loger au moins partiellement le raccord mâle 10 et le raccord femelle 20 et les maintenir en position.

Le carter 30 peut notamment être emmanché sur le raccord mâle 10 et le raccord femelle 20, de manière à autoriser leur rotation relative autour de l'axe longitudinal X, tout en limitant leur déplacement le long de l'axe longitudinal X.

Afin d'empêcher tout dévissage accidentel du système de connexion médicale 1, le carter 30 comprend en outre des moyens de blocage en rotation du raccord mâle 10 et du raccord femelle 20 autour de l'axe longitudinal X dans le sens de vissage de leur embout fileté respectif.

Plus précisément, les moyens de blocage sont capables de bloquer en rotation le raccord mâle 10 dans le sens de vissage de son embout distal uniquement, et en parallèle le raccord femelle 20 dans le sens de vissage de son embout proximal uniquement, le sens de vissage de l'embout distal 11 du raccord mâle 10 étant opposé au sens de vissage de l'embout proximal 22 du raccord femelle 20. De la sorte, il est possible de visser sur chaque embout fileté un dispositif médical en bloquant embout par rapport au carter 30, mais pas de le dévisser, puisque l'embout est libre en rotation dans le carter 30.

Par ailleurs, afin d'empêcher un opérateur de bloquer en rotation l'un ou l'autre des raccords 10, 20 pour pouvoir dévisser le dispositif de l'embout fileté 11, 22 correspondant, aucun des raccords 10, 20 n'est accessible sans démonter le système de connexion 1, et ne peut donc être saisi par l'opérateur puisqu'ils sont tous deux logés en majeure partie dans le carter 30.

Dans les formes de réalisation illustrées sur les figures, les moyens de blocage en rotation du carter 30 comprennent chacun au moins une encoche 33, adaptée pour coopérer avec une dent 15, 25 réalisée dans le raccord 10, 20 correspondant.

Ici, les moyens de blocage comprennent chacun deux dents 15, 25, s'étendant depuis le raccord 10, 20 correspondant, et au moins autant d'encoche 33, par exemple quatre pour chaque raccord 10, 20.

Pour cela, le carter 30 comprend des renfoncements 31, 32 dans sa paroi interne, au niveau de ses extrémités proximale et distale, et les raccords mâle 10 et femelle 20 comprennent une surépaisseur 15a, 25a correspondante au niveau de l'extrémité de leur raccord distal 11 et proximal 21 respectivement.

La surépaisseur 15a du raccord mâle 10 présente une forme globalement tubulaire, faisant saillie le long de la périphérie de l'embout distal 11 du raccord mâle 10, de laquelle s'étend au moins une dent 15, ici deux dents, en direction de l'embout proximal 12 du raccord 10. Chaque dent 15 présente une arête de blocage 15b sensiblement parallèle à l'axe longitudinal X et une arête de guidage 15c, s'étendant de manière continue entre une première extrémité libre de l'arête de blocage 15b et la surépaisseur 15a.

De manière similaire, la surépaisseur 25a du raccord femelle 20 présente une forme globalement tubulaire, faisant saillie le long de la périphérie de l'embout proximal 22 du raccord femelle 20, de laquelle s'étend au moins une dent 25, ici deux dents 25, en direction de l'embout distal 21 du raccord 20. Chaque dent 25 présente une arête de blocage 25b sensiblement parallèle à l'axe longitudinal X et une arête de guidage 25c, s'étendant de manière continue entre une première extrémité libre de l'arête de blocage 25a et la surépaisseur 25a.

Chaque dent 15, 25 peut donc être formée intégralement avec la surépaisseur 15a, 25a, elle-même étant monobloc avec le reste du raccord 10, 20 correspondant.

Par ailleurs, chaque renfoncement 31, 32 du carter 30 est de forme globalement annulaire, et est délimité par un bord 33a adapté pour venir en butée contre la surépaisseur 15a, 25a en regard (c'est-à-dire la surépaisseur 15a du raccord mâle 10 dans le cas du renfoncement distal 31, et la surépaisseur 25a du raccord femelle 20 dans le cas du renforcement proximal 32). Le bord 33a du renfoncement 31, 32 définit une encoche 33, de forme triangulaire complémentaire de la forme des dents 15, 25, et adaptée pour recevoir éventuellement une dent 15, 25 lors de la connexion d'un dispositif médical au système de connexion médicale 1. Plus précisément, l'encoche 33 est formée d'une arête de blocage 33b, sensiblement parallèle à l'axe longitudinal X, et d'une arête de guidage 33c, s'étendant de manière continue entre une extrémité de l'arête de blocage 33b et le bord 33a du renfoncement 31, 32, le long de la périphérie du carter 30.

Lorsque le renfoncement 31, 32 du carter 30 comprend quatre encoche 33, l'arête de guidage 33b d'une encoche 33 donnée peut par exemple s'étendre entre l'extrémité libre de l'arête de blocage 33c correspondante et la base de l'arête de blocage 33b de l'encoche 33 adjacente.

Un diamètre interne de la paroi interne du carter 30 au niveau des renfoncements proximal 32 et distal 31 est en outre globalement égal au diamètre externe des surépaisseurs 15a, 25a des raccords mâle 10 et femelle 20, afin de garantir leur mise en prise lors d'une éventuelle connexion. Par ailleurs, le diamètre interne de la paroi interne du carter 30 dans la zone centrale 34 située entre les deux renfoncements 31, 32 est plus faible, et est au moins égal au diamètre externe de l'embout distal 11 du raccord mâle 10 et de l'embout proximal 22 du raccord femelle 20.

De la sorte, lorsque le carter 30 est emmanché sur le raccord mâle 10 et le raccord femelle 20, et que le raccord mâle 10 et le raccord femelle 20 sont accouplés, il n'est plus possible de les extraire du carter 30. En effet, les bords des renfoncements 31, 32, qui délimitent la zone centrale 34 de la paroi interne du carter 30, viennent en butée contre les surépaisseurs 15a, 25a des raccords 10, 20, qui empêchent ainsi leur extraction.

Les dimensions, et notamment la longueur du carter 30 est par ailleurs choisie de sorte que, lorsqu'il loge le raccord mâle 10 et le raccord femelle 20 et que les raccords 10, 20 sont accouplés, seul leurs embouts filetés respectifs 11, 22 sont éventuellement susceptibles de faire saillie en dehors du carter 30, ces embouts 11, 22 étant destinés à être dissimulés dans les embouts correspondants des dispositifs médicaux lors de leur connexion à ces dispositifs. De cette manière, le carter 30 est la seule pièce du système de connexion 1 pouvant être manipulée par l'opérateur. Il peut donc comprendre des moyens de préhension améliorant la manipulation du carter 30 par l'opérateur, formés par exemple d'empreintes concaves 35 réalisées dans sa surface externe, et/ou de rainures longitudinales 36, faisant saillie de la paroi externe.

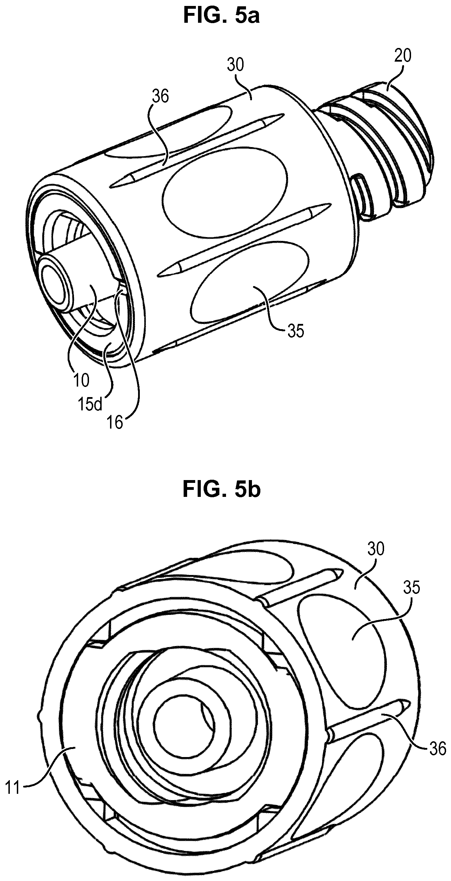

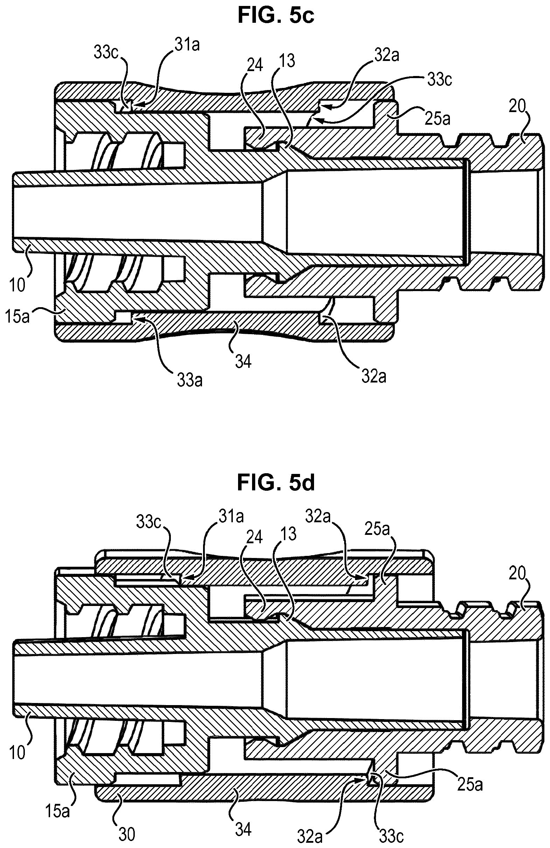

Dans la forme de réalisation illustrée sur les figures 5a à 5c, le carter 30 loge l'intégralité de l'embout distal 11 du raccord mâle 10 et de l'embout proximal 22 du raccord femelle 20, seuls les filets de l'embout proximal 22 et éventuellement la partie tubulaire de l'embout distal 11 n'étant pas recouverts. Toutefois, on remarquera que, lorsque le système de connexion médicale 1 est connecté à des dispositifs médicaux, l'embout proximal 22 du raccord femelle 20 est connecté à un embout mâle, ici de type Luer Lock®, et donc recouvert par la collerette filetée complémentaire de cet embout mâle, tandis que l'embout distal 11 du raccord mâle 10 est connecté à un embout femelle, également de type Luer Lock®. L'embout proximal 22 n'est donc plus accessible. La collerette de l'embout distal 11 quant à elle peut être dissimulée par le carter 30, s'il est suffisamment long pour la recouvrir, ce qui empêche un opérateur de la maintenir fixe en rotation afin de dévisser le dispositif qui y est connecté.

De préférence, les arêtes de guidage 33c des encoches 33 du renfoncement distal 31 et celles du renfoncement proximal 32 s'étendent sensiblement parallèlement. Le creux des encoches 33 du renfoncement proximal 32 sont donc opposés aux creux des encoches 33 du renfoncement distal 31 du carter 30.

Le système de connexion médicale 1 comprend alors, lorsque l'ensemble des pièces qui le composent sont assemblées, le raccord mâle 10 et le raccord femelle 20, accouplés de manière à être mobile en rotation l'un par rapport à l'autre, et sensiblement fixes en translation, logés dans le carter 30.

Pour cela, le carter 30 peut par exemple être emmanché sur l'un des raccords 10, 20, disons le raccord femelle 20, en positionnant les dents 25 du raccord femelle 20 en regard des encoches 33 correspondantes du carter 30. Le raccord femelle 20 est de préférence positionné en butée dans le carter 30, en insérant les dents 25 du raccord 20 dans des encoches 33 correspondantes du carter 30.

Le raccord mâle 10 peut alors être introduit dans le carter 30 et le raccord femelle 20. Pour cela, l'embout proximal 12 du raccord mâle 10 est introduit progressivement dans le carter 30, par son extrémité distale, de telle manière que son embout proximal 12 pénètre dans l'embout distal 21 du raccord femelle 20. Lorsque la collerette 14 de l'embout distal 11 du raccord mâle 10 vient en butée contre l'appui 23 de l'embout proximal 22 du raccord femelle 20, il suffit alors d'exercer un léger effort sur les raccords 10, 20 pour que la collerette 14 écarte l'appui 23 et qu'il vienne se loger derrière la collerette 14. Le raccord mâle 10 et le raccord femelle 20 sont alors accouplés par la coopération de leurs moyens d'encliquetage 14, 23 au sein du carter 30.

De manière alternative, le carter 30 est d'abord emmanché sur le raccord mâle 10, puis le raccord femelle 20 est positionné en l'insérant par l'extrémité proximale du carter 30.

Il n'est alors plus possible de démonter les pièces du système de connexion 1. En effet, comme indiqué plus haut, les moyens d'encliquetage 14, 23 du raccord mâle 10 et du raccord femelle 20 empêchent toute séparation des raccords 10, 20. Par ailleurs, le diamètre interne de la zone centrale 34 du carter 30 étant plus faible que celui des surépaisseurs 15a, 25a des raccords 10, 20 qui portent les dents 15, 25, aucun des deux raccords 10, 20 n'est susceptible de passer, ces derniers étant bloqués par les bords 31a, 32a des renfoncements 31, 32 du carter 30.

Afin de faciliter l'introduction du raccord mâle 10 dans le raccord femelle 20 et d'éviter d'abimer les pointes des dents 15, 25 des raccords 10, 20, le raccord femelle 20 et le raccord mâle 10 peuvent être munis de repères visuels 16, 26 servant d'aide au positionnement relatif du raccord mâle 10 et du raccord femelle 20 par rapport au carter 30. Par exemple, les raccords 10, 20 peuvent présenter des sillons 16, 26, réalisés dans la surface 15d, 25d de la surépaisseur 15a, 25a qui est opposée aux dents 15, 25. Les sillons 16, 26 peuvent notamment être disposé sur la surface 15d, 25d par rapport aux dents 15, 25 de sorte qu'il suffit à l'opérateur de placer les sillons 16 du raccord mâle 10 en regard des sillons 26 du raccord femelle 20 lors de l'introduction du raccord mâle 10 dans le carter 30 et le raccord femelle 20. En effet, le raccord femelle 20 étant déjà introduit dans le carter 30 de manière fixe, et de préférence dans les encoches 33 du renfoncement proximal 32, en positionnant le raccord mâle 10 par rapport au raccord femelle 20, celui-ci est également positionné par rapport au carter 30.

Le système de connexion médicale 1 est maintenant prêt à être utilisé. Si un opérateur souhaite mettre en communication sans risque de dévissages intempestifs des dispositifs médicaux, il lui suffit alors de les visser de part et d'autre du système 1.

Par exemple, il peut visser un premier dispositif muni d'un embout Luer Lock® mâle sur l'embout proximal 22 du raccord femelle 20 (qui est ici un embout Luer Lock® femelle). Au cours de cette manipulation, l'opérateur saisi le système de connexion 1 par son carter 30, et positionne l'embout mâle dans l'embout femelle 22 du raccord femelle 20. Il est alors naturellement amené à pousser sur l'embout mâle et le raccord 20, ce qui amène le raccord femelle 20 en butée contre le bord 32a du renfoncement 32 proximal 32 du carter 30 (figure 5d). La forme particulière du renfoncement 32 guide le raccord femelle 20, grâce aux arêtes de guidage 25c, 33c des dents 25 et des encoches 33 en regard et font tourner le raccord 20 par rapport au carter 30 jusqu'à ce que les dents 25 pénètrent dans des encoches 33. Les arêtes de blocage 25b des dents viennent alors en butée contre les arêtes de blocage 33b des encoches 33, stoppant nette la rotation du raccord femelle 20 par rapport au carter 30. En poursuivant le mouvement de rotation de l'embout mâle du dispositif médical par rapport au carter 30, l'opérateur peut alors visser l'embout mâle sur l'embout femelle 22 du raccord femelle 20, qui est à présent fixe en rotation par rapport au carter 30.

Il en est bien entendu de même pour la connexion d'un deuxième dispositif, muni cette fois d'un embout Luer Lock femelle sur l'embout distal 11 du raccord mâle 10 (qui est ici un embout Luer Lock mâle). Au cours de cette manipulation, l'opérateur saisi le système de connexion 1, qui peut déjà être fixé au premier dispositif médical, par le carter 30, positionne l'embout femelle dans l'embout mâle 11 du raccord mâle 10, et pousse sur l'embout femelle vers le raccord 10. Le raccord mâle 10 est donc déplacé le long de l'axe longitudinal X jusqu'à ce qu'il arrive en butée contre le bord 31a du renfoncement distal 31 du carter 30. On notera que c'est l'ensemble formé du raccord mâle 10 accouplé au raccord femelle 20 qui se déplace dans le carter 30, étant donné que leurs moyens d'encliquetage 14, 23 n'autorise pas de translation relative substantielle. La forme particulière du renfoncement 31 guide le raccord mâle 10, grâce aux arêtes de guidage 15c, 33c des dents 15 et des encoches 33 en regard, jusqu'à ce que les dents 15 pénètrent dans des encoches 33. Les arêtes de blocage 15b des dents 15 viennent alors en butée contre les arêtes de blocage 33b des encoches 33, stoppant nette la rotation du raccord mâle 10 par rapport au carter 30. En poursuivant le mouvement de rotation de l'embout femelle du deuxième dispositif médical par rapport au carter 30, il peut alors visser l'embout femelle sur l'embout mâle 11 du raccord mâle 10, qui est à présent fixe en rotation par rapport au carter 30.

Une fois l'embout d'un dispositif vissé sur l'un des embouts 11, 22 correspondants du système de connexion 1, par exemple l'embout 22 du raccord femelle 20, il n'est alors plus possible de le dévisser, même si l'autre embout 11 est libre (et qu'aucun dispositif n'y est fixé). En effet, la rotation du raccord 20 par rapport au carter 30 dans le sens horaire, qui, dans les exemples illustrés sur les figures, est le sens de vissage, est bloquée par la butée des arêtes de blocage 33b des encoches 33 contre celles des dents 25 du raccord femelle 20. Cette rotation ne fait par conséquent que renforcer le vissage du dispositif sur l'embout 22. Dans le sens contraire, c'est-à-dire dans le sens de dévissage, les arêtes de guidage 25c des dents 25 glissent sur les arêtes de guidage 33c des encoches 33, sans pouvoir venir en prise. Etant donné qu'il n'est pas possible de fixer le raccord 20 en rotation dans ce sens, puisqu'il est complètement recouvert par le carter 30 et/ou l'embout du dispositif médical, et qu'il est monté à rotation sur l'autre raccord 10, il est donc bien impossible de déconnecter le dispositif médical ou de démonter le système de connexion 1 sans les endommager.

Selon une forme de réalisation (non illustrée sur les figures), l'embout du raccord mâle 10 et celui du raccord femelle 20 peuvent être de types différents (tout en restant de préférence des embouts vissables). Le système de connexion 1 autorise par conséquent la connexion de deux dispositifs ayant des moyens de connexion différents, et peut ainsi servir de manière avantageuse d'adaptateur de connexion.

Par ailleurs, on comprendra qu'il est désormais plus simple pour un fabricant de proposer des circuits déjà montés, comprenant par exemple deux dispositifs médicaux connectés par l'intermédiaire d'un système de connexion 1 conforme à l'invention, sans risquer de dévissage accidentel, quelles que soient les conditions de transport ou d'utilisation du circuit.

Enfin, l'utilisation d'un raccord mâle 10 et d'un raccord femelle 20 dont l'accouplement est étanche, comme c'est le cas ici notamment grâce à la surépaisseur d'étanchéité 13 et la forme complémentaires des embouts 12, 21, permet d'obtenir un système de connexion médicale 1 capable de réaliser une connexion fluidique étanche.

Selon une autre forme de réalisation, le système de connexion médicale 1 peut ne comprendre qu'un seul raccord, mâle ou femelle. Dans ce cas, le système de connexion comprend alors des moyens de retenue en translation du raccord dans le carter, capables de limiter les déplacements du raccord dans le carter le long de l'axe longitudinal X, tout en autorisant leur rotation par rapport au carter. En effet, afin de permettre aux dents de glisser sur les arêtes de guidage des encoches lorsque l'on fait tourner le raccord dans son sens de dévissage, il est nécessaire que le raccord puisse se déplacer le long de l'axe longitudinal X pour que les dents ne puissent pas venir en prise contre les arêtes de blocage. Le raccord doit cependant être retenu dans le carter si l'on souhaite empêcher l'opérateur de saisir le raccord, dans la mesure où cela permettrait de le dévisser.

Dans le système décrit en référence aux figures annexées, le rôle des moyens de retenue est joué par le deuxième raccord. En effet, les moyens d'encliquetage des raccords autorisent leur rotation relative, et la collaboration de la surépaisseur du raccord avec le renfoncement correspondant du carter permet de limiter les translations des raccords le long de l'axe longitudinal X.

En l'absence de deuxième raccord, notamment lorsque le système de connexion est directement fixé, par collage, soudage, ou autre moyen de fixation, ou est venu de matière avec un dispositif médical, les moyens de retenue peuvent comprendre des moyens d'encliquetage formés d'une collerette et d'un appui, adaptés pour coopérer de manière similaire à ce qui a été décrit précédemment, l'appui pouvant glisser sur une distance sensiblement égale à la longueur des arêtes de blocage des dents du raccord une fois les moyens d'encliquetage accouplés.

Le fonctionnement du système est alors identique à ce qui a été décrit plus haut, seul un dispositif médical pouvant être connecté, sans dévissage intempestif, au système de connexion médicale 1 ainsi obtenu.

## Revendications

1. Système de connexion médicale (1)comprenant en combinaison :
- un premier raccord (10) définissant un passage coaxial avec un axe longitudinal (X), comprenant un premier embout fileté (11),
- un carter (30), coaxial à l'axe longitudinal (X), adapté pour loger au moins partiellement le premier raccord (10), de sorte que le premier raccord (10) soit mobile en rotation autour de l'axe longitudinal (X) dans le carter (30), et
- un deuxième raccord (20), définissant un passage coaxial avec le premier raccord (10) et comprenant un deuxième embout fileté (22), ledit deuxième raccord étant adapté pour être logé dans le carter (30), le premier raccord (10) et le deuxième raccord (20) étant mobiles en rotation l'un par rapport à l'autre autour de l'axe longitudinal (X) dans le carter (30) le système étant **caractérisé en ce que** :
- le carter (30) comprend en outre des moyens de retenue en translation du premier raccord, aptes à limiter le déplacement relatif du premier raccord par rapport au carter le long de l'axe longitudinal, des moyens de blocage en rotation (33) du premier raccord (10) autour de l'axe longitudinal (X) seulement dans le sens de vissage du premier embout fileté (11) du premier raccord (10) et des moyens de blocage en rotation (33) du deuxième raccord (20) autour de l'axe longitudinal (X) seulement dans le sens de vissage du deuxième embout fileté (22) du deuxième raccord (20), le sens de vissage du premier embout fileté (11) du premier raccord (10) étant opposé au sens de vissage du deuxième embout fileté (22) du deuxième raccord (20)
- le deuxième raccord est fixé sur le premier raccord (10) de manière à bloquer le premier raccord (10) en translation par rapport au carter (30) et
- le premier raccord (10) comprend un premier embout de connexion (12) et le deuxième raccord (20) comprend un deuxième embout de connexion (21), le premier embout de connexion (12) étant connecté à l'aide de moyens d'encliquetage (14, 23) au deuxième embout de connexion (21) de sorte que le premier raccord (10) reste mobile en rotation autour de l'axe longitudinal (X) par rapport au deuxième raccord (20).

2. Système de connexion médicale (1) selon la revendication 1, dans lequel les moyens de blocage en rotation comprennent au moins une encoche (33) réalisée dans une paroi interne du carter (30), adaptée pour coopérer avec une dent (15) s'étendant depuis le premier raccord (10).

3. Système de connexion médicale (1) selon la revendication 2, dans lequel la ou chaque dent (15) fait saillie le long de l'axe longitudinal (X) d'une surépaisseur annulaire (15a) s'étendant le long d'une périphérie de l'embout fileté (11), et présente une première arête (15b) sensiblement parallèle à l'axe longitudinal (X) et une seconde arête (15c), s'étendant de manière continue entre une première extrémité libre de la première arête (15b) et la surépaisseur (15a).

4. Système de connexion médicale (1) selon l'une des revendications 2 ou 3, dans lequel la ou chaque encoche (33) est définie par un renfoncement (31, 32) annulaire de la paroi interne du carter (30) qui s'étend transversalement par rapport à l'axe longitudinal (X), et comprend une première arête (33b) sensiblement parallèle à l'axe longitudinal (X) et une deuxième arête (33c), s'étendant de manière continue entre une extrémité de la première arête (33b) et un bord annulaire (31a, 32a) dudit renfoncement (31,32).

5. Système de connexion médicale (1) selon l'une des revendications 1 à 4, dans lequel le carter (30) est dimensionné de sorte que, lorsque le premier raccord (10) et le deuxième raccord (20) sont logés dans le carter (30), seul l'embout fileté du premier raccord (10) et du deuxième raccord est susceptible de faire saillie en dehors du carter (30), de sorte que le premier raccord (10) et le deuxième raccord (20) ne présentent pas de surface susceptible d'être saisie par un opérateur.

6. Système de connexion médicale (1) selon l'une des revendications 1 à 5, dans lequel le premier embout de connexion (12) est adapté pour être inséré dans le deuxième embout de connexion (21), et les moyens d'encliquetage (14, 23) comprennent une collerette (14) et un appui (23) en regard s'étendant respectivement du premier embout de connexion (12) et du deuxième embout de connexion (21), adaptés pour venir en prise lorsque le premier embout de connexion (12) est inséré dans le deuxième embout de connexion (21).

7. Système de connexion médicale (1) selon l'une des revendications 1 à 6, dans lequel le premier raccord (10) comprend un premier embout de connexion (12) adapté pour être inséré dans un deuxième embout de connexion (21) du deuxième raccord (20), et le système de connexion (1) comprend en outre un organe d'étanchéité (13) disposé entre le premier embout de connexion (12) et le deuxième embout de connexion (21).

8. Système de connexion médicale (1) selon l'une des revendications 1 à 7, dans lequel le premier raccord (10) et le deuxième raccord (20) comprennent des repères visuels (16, 26) servant d'aide au positionnement relatif du raccord mâle (10) et du raccord femelle (20) par rapport au carter (30).

## Patentansprüche

1. Medizinisches Verbindungssystem (1), umfassend in Kombination:
- einen ersten Anschluss (10), der einen mit einer Längsachse (X) koaxialen Durchgang definiert, umfassend einen ersten Gewindeansatz (11),
- ein zu der Längsachse (X) koaxiales Gehäuse (30), das geeignet ist, den ersten Anschluss (10) mindestens teilweise unterzubringen, so dass der erste Anschluss (10) in dem Gehäuse (30) um die Längsachse (X) rotatorisch beweglich ist, und
- einen zweiten Anschluss (20), der mit dem ersten Anschluss (10) einen koaxialen Durchgang definiert und einen zweiten Gewindeansatz (22) umfasst, wobei der zweite Anschluss geeignet ist, in dem Gehäuse (30) untergebracht zu sein, wobei der erste Anschluss (10) und der zweite Anschluss (20) in dem Gehäuse (30) in Bezug aufeinander um die Längsachse (X) rotatorisch beweglich sind,
wobei das System **dadurch gekennzeichnet ist, dass**
- das Gehäuse (30) ferner Translationsrückhaltemittel des ersten Anschlusses umfasst, die imstande sind, die relative Verlagerung des ersten Anschlusses in Bezug auf das Gehäuse entlang der Längsachse, der Rotationsblockiermittel (33) des ersten Anschlusses (10) um die Längsachse (X) nur in Schraubrichtung des ersten Gewindeansatzes (11) des ersten Anschlusses (10) und der Rotationsblockiermittel (33) des zweiten Anschlusses (20) um die Längsachse (X) nur in Schraubrichtung des zweiten Gewindeansatzes (22) des zweiten Anschlusses (20) zu begrenzen, wobei die Schraubrichtung des ersten Gewindeansatzes (11) des ersten Anschlusses (10) entgegengesetzt zur Schraubrichtung des zweiten Gewindeansatzes (22) des zweiten Anschlusses (20) ist,
- der zweite Anschluss auf dem ersten Anschluss (10) derart befestigt ist, dass der erste Anschluss (10) in Bezug auf das Gehäuse (30) translatorisch blockiert wird, und
- der erste Anschluss (10) einen ersten Verbindungsansatz (12) umfasst und der zweite Anschluss (20) einen zweiten Verbindungsansatz (21) umfasst, wobei der erste Verbindungsansatz (12) mit Hilfe von Rastmitteln (14, 23) mit dem zweiten Verbindungsansatz (21) verbunden ist, so dass der erste Anschluss (10) um die Längsachse (X) in Bezug auf den zweiten Anschluss (20) rotatorisch beweglich bleibt.

2. Medizinisches Verbindungssystem (1) nach Anspruch 1, wobei die Rotationsblockiermittel mindestens eine Kerbe (33) umfassen, die in einer Innenwand des Gehäuses (30) realisiert ist, die zum Zusammenwirken mit einem Zahn (15) geeignet ist, der sich ab dem ersten Anschluss (10) erstreckt.

3. Medizinisches Verbindungssystem (1) nach Anspruch 2, wobei der oder jeder Zahn (15) entlang der Längsachse (X) von einer ringförmigen Überdicke (15a) hervorsteht, die sich entlang eines Umfangs des Gewindeansatzes (11) erstreckt und eine erste Kante (15b) aufweist, die etwa parallel zur Längsachse (X) ist, und eine zweite Kante (15c), die sich kontinuierlich zwischen einem ersten freien Ende der ersten Kante (15b) und der Überdicke (15a) erstreckt.

4. Medizinisches Verbindungssystem (1) nach einem der Ansprüche 2 oder 3, wobei die oder jede Kerbe (33) von einer ringförmigen Vertiefung (31, 32) der Innenwand des Gehäuses (30) definiert ist, die sich transversal in Bezug zur Längsachse (X) erstreckt und eine erste Kante (33b) umfasst, die etwa parallel zur Längsachse (X) ist, und eine zweite Kante (33c), die sich kontinuierlich zwischen einem Ende der ersten Kante (33b) und einem ringförmigen Rand (31a, 32a) der Vertiefung (31, 32) erstreckt.

5. Medizinisches Verbindungssystem (1) nach einem der Ansprüche 1 bis 4, wobei das Gehäuse (30) derart dimensioniert ist, dass, wenn der erste Anschluss (10) und der zweite Anschluss (20) in dem Gehäuse (30) untergebracht sind, nur der Gewindeansatz des ersten Anschlusses (10) und des zweiten Anschlusses imstande ist, aus dem Gehäuse (30) hervorzustehen, so dass der erste Anschluss (10) und der zweite Anschluss (20) keine Fläche aufweisen, die imstande ist, von einem Bediener ergriffen zu werden.

6. Medizinisches Verbindungssystem (1) nach einem der Ansprüche 1 bis 5, wobei erste Verbindungsansatz (12) geeignet ist, in den zweiten Verbindungsansatz (21) eingesetzt zu sein, und die Rastmittel (14, 23) einen Ring (14) und eine zugewandte Abstützung (23) umfassen, die sich jeweils vom ersten Verbindungsansatz (12) und vom zweiten Verbindungsansatz (21) erstrecken, die geeignet sind, in Eingriff zu kommen, wenn der erste Verbindungsansatz (12) in den zweiten Verbindungsansatz (21) eingesetzt ist.

7. Medizinisches Verbindungssystem (1) nach einem der Ansprüche 1 bis 6, wobei der erste Anschluss (10) einen ersten Verbindungsansatz (12) umfasst, der geeignet ist, in einen zweiten Verbindungsansatz (21) des zweiten Anschlusses (20) eingesetzt zu sein, und das Verbindungssystem (1) ferner ein Dichtungsorgan (13) umfasst, das zwischen dem ersten Verbindungsansatz (12) und dem zweiten Verbindungsansatz (21) angeordnet ist.

8. Medizinisches Verbindungssystem (1) nach einem der Ansprüche 1 bis 7, wobei der erste Anschluss (10) und der zweite Anschluss (20) visuelle Markierungen (16, 26) umfassen, die als Hilfe für die relative Positionierung des männlichen Anschlusses (10) und des weiblichen Anschlusses (20) in Bezug auf das Gehäuse (30) dienen.

## Claims

1. Medical connection system (1) comprising in combination:
- a first coupling (10) defining a passage coaxial with a longitudinal axis (X), comprising a first threaded end (11),
- a casing (30), coaxial with the longitudinal axis (X), adapted to house at least partially the first coupling (10), such that the first coupling (10) is rotatable about the longitudinal axis (X) in the casing (30), and
- a second coupling (20), defining a passage coaxial with the first coupling (10) and comprising a second threaded end (22), said second coupling being adapted to be housed in the casing (30), the first coupling (10) and the second coupling (20) being rotatable in relation to one another about the longitudinal axis (X) in the casing (30)
the system being **characterised in that**:
- the casing (30) further comprises means for retaining the first coupling in translation, capable of limiting the relative movement of the first coupling with respect to the casing along the longitudinal axis, means for locking in rotation (33) the first coupling (10) about the longitudinal axis (X) only in the screwing direction of the first threaded end (11) of the first coupling (10) and means for locking in rotation (33) the second coupling (20) about the longitudinal axis (X) only in the screwing direction of the second threaded end (22) of the second coupling (20), the screwing direction of the first threaded end (11) of the first coupling (10) being opposite the screwing direction of the second threaded end (22) of the second coupling (20)
- the second coupling is attached to the first coupling (10) so as to lock the first coupling (10) in translation with respect to the casing (30) and
- the first coupling (10) comprises a first connection end (12) and the second coupling (20) comprises a second connection end (21), the first connection end (12) being connected using latching means (14, 23) to the second connection end (21) such that the first coupling (10) remains rotatable about the longitudinal axis (X) with respect to the second coupling (20).

2. Medical connection system (1) according to claim 1, wherein the means for locking in rotation comprise at least one notch (33) made in an inner wall of the casing (30), adapted to cooperate with a tooth (15) extending from the first coupling (10).

3. Medical connection system (1) according to claim 2, wherein the or each tooth (15) protrudes along the longitudinal axis (X) of an annular extra thickness (15a) extending along a periphery of the threaded end (11), and has a first edge (15b) substantially parallel with the longitudinal axis (X) and a second edge (15c), extending continuously between a first free end of the first edge (15b) and the extra thickness (15a).

4. Medical connection system (1) according to one of claims 2 or 3, wherein the or each notch (33) is defined by an annular recess (31, 32) of the inner wall of the casing (30) which extends transversally with respect to the longitudinal axis (X), and comprises a first edge (33b) substantially parallel to the longitudinal axis (X) and a second edge (33c) extending continuously between one end of the first edge (33b) and an annular edge (31a, 32a) of said recess (31, 32).

5. Medical connection system (1) according to one of claims 1 to 4, wherein the casing (30) is sized such that, when the first coupling (10) and the second coupling (20) are housed in the casing (30), only the threaded end of the first coupling (10) and of the second coupling is capable of protruding out of the casing (30), such that the first coupling (10) and the second coupling (20) do not have a surface capable of being gripped by an operator.

6. Medical connection system (1) according to one of claims 1 to 5, wherein the first connection end (12) is adapted to be inserted into the second connection end (21), and the latching means (14, 23) comprise a flange (14) and a facing support (23) extending respectively from the first connection end (12) and from the second connection end (21), adapted to engage when the first connection end (12) is inserted into the second connection end (21).

7. Medical connection system (1) according to one of claims 1 to 6, wherein the first coupling (10) comprises a first connection end (12) adapted to be inserted into a second connection end (21) of the second coupling (20), and the connection system (1) further comprises a sealing member (13) disposed between the first connection end (12) and the second connection end (21).

8. Medical connection system (1) according to one of claims 1 to 7, wherein the first coupling (10) and the second coupling (20) comprise visual markers (16, 26) serving to assist the relative positioning of the male coupling (10) and the female coupling (20) with respect to the casing (30).
